# EUROPEAN PATENT APPLICATION

(11) **EP 4 796 527 A1**
(43) Date of publication of application: **26.08.2026**
(21) Application number: 24879922.3
(22) Date of filing: 20.08.2024
(51) Int. Cl.: C07C 219/06, C07C 233/36, C07C 229/16, C07C 211/63, C07C 237/04, C07C 271/14, C07C 323/25, C07F 9/40, C07C 213/08, A61K 47/54

(54) **CATIONIC LIPID AND METHOD FOR PREPARING SAME**

(30) Priority: 19.10.2023 KR 20230140257
(71) Applicant: Samyang Biopharmaceuticals Corporation, Seongnam-si Gyeonggi-do 13488 (KR)
(72) Inventor: PARK, Jong Min, Hwaseong-si Gyeonggi-do 18484 (KR); SEONG, Shi Hwa, Seoul 06008 (KR); KIM, Sol, Seongnam-si Gyeonggi-do 13488 (KR); NAM, Joung Pyo, Seoul 02471 (KR)
(74) Representative: Gleiss Große Schrell und Partner mbB
(86) International application number: PCT/KR2024/012368
(87) International publication number: WO 2025/084582

(57) **Abstract**

The present invention relates to a cationic lipid and a method for preparing same, and more specifically to: a cationic lipid which, due to the specific structure thereof, forms (or is capable of forming) a complex with an anionic drug, and thus is useful for drug delivery; and a method for preparing same.

## Description

### TECHNICAL FIELD

The present invention relates to a cationic lipid and a method for preparing the same, and more specifically, the present invention relates to a cationic lipid which can easily form a complex with anionic drug due to its specific structure, and thus is useful for drug delivery, and a method for preparing the same.

### BACKGROUND ART

In therapies using anionic drugs including nucleic acid, technologies for safe and efficient drug delivery have been researched for a long time, and various carriers and techniques for delivery have been developed. Carriers are mainly divided into viral carriers utilizing adenovirus, retrovirus or the like, and non-viral carriers utilizing cationic lipid, cationic polymer or the like. Viral carriers are known as being exposed to risks such as non-specific immune response, etc. and having many problems in commercialization due to the complexity of the production process. Thus, recent researches proceed in the direction to improve such disadvantages by using non-viral carriers. In comparison with viral carriers, non-viral carriers have advantages of fewer side effects in terms of *in vivo* safety, and lower production cost in terms of economy.

The representative non-viral carriers for delivering nucleic acid material are a complex of cationic lipid and nucleic acid (lipoplex) and a complex of polycationic polymer and nucleic acid (polyplex). Such a cationic lipid or polycationic polymer stabilizes anionic drug by forming a complex through electrostatic interaction with the anionic drug and increases intracellular delivery, and for these reasons, various researches thereof have been conducted (De Paula D, Bentley MV, Mahato RI, Hydrophobization and bioconjugation for enhanced siRNA delivery and targeting, RNA 13 (2007) 431-56; Gary DJ, Puri N, Won YY, Polymer-based siRNA delivery: Perspectives on the fundamental and phenomenological distinctions from polymer-based DNA delivery, J Control release 121 (2007) 64-73).

However, polycation polymers have cytotoxicity due to their multivalent cationic charges, making them problematic for practical use, and nucleic acid-cationic lipid complexes have low stability in the blood, making them difficult to use for actual *in vivo* applications. In addition, ionic liposomes containing cationic lipid, neutral lipid, and fusogenic lipid have disadvantages such as the complex synthesis method of the cationic lipid used, cytotoxicity, and low efficiency of intracellular delivery of nucleic acid.

### CONTENTS OF THE INVENTION

### PROBLEMS TO BE SOLVED

The purpose of the present invention is to provide a cationic lipid having a specific structure which can easily form a complex with anionic drug and thus is useful for drug delivery, and a method for preparing the same.

### TECHNICAL MEANS

The first aspect of the present invention provides a cationic lipid having a structure represented by the following formula 1: 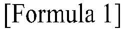 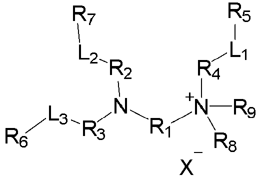 wherein, in the above formula 1,
R₁ is a substituted or unsubstituted alkylene group,
each of R₂, R₃ and R₄ is independently a substituted or unsubstituted alkylene group,
each of R₅, R₆ and R₇ is independently a substituted or unsubstituted, saturated or unsaturated monovalent hydrocarbon group,
each of R₈ and R₉ is independently a substituted or unsubstituted alkyl group or carbocyclic group, or is independently -R₁₀-(L₄)ₙ-R₁₁,
each R₁₀ is independently a substituted or unsubstituted alkylene group,
each R₁₁ is independently a substituted or unsubstituted, saturated or unsaturated monovalent hydrocarbon group,
each of L₁, L₂, L₃ and L₄ is independently selected from the group consisting of -C(O)O-, -OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, alkenylene, arylene, and heteroarylene, wherein L' is a direct bond, alkylene or alkenylene, and each R' is independently selected from the group consisting of hydrogen atom, alkyl and alkenyl,
n is 0 or 1, and
X⁻ is a pharmaceutically acceptable monovalent anion.

More specifically, in the above formula 1,
R₁ is a substituted or unsubstituted C₁₋₆ alkylene group,
each of R₂, R₃ and R₄ is independently a substituted or unsubstituted C₃₋₁₂ alkylene group,
each of R₅, R₆ and R₇ is independently a substituted or unsubstituted, saturated or unsaturated monovalent C₃₋₂₀ hydrocarbon group,
each of R₈ and R₉ is independently a substituted or unsubstituted C₁₋₆ alkyl group or C₃₋₆ carbocyclic group, or is independently -R₁₀-(L₄)ₙ-R₁₁,
each R₁₀ is independently a substituted or unsubstituted C₃₋₁₂ alkylene group,
each R₁₁ is independently a substituted or unsubstituted, saturated or unsaturated monovalent C₃₋₂₀ hydrocarbon group,
each of L₁, L₂, L₃ and L₄ is independently selected from the group consisting of -C(O)O-, -OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, C₂₋₆ alkenylene, C₆₋₂₀ arylene, and C₃₋₂₀ heteroarylene, wherein L' is a direct bond, C₁₋₁₃ alkylene or C₂₋₁₃ alkenylene, and each R' is independently selected from the group consisting of hydrogen atom, C₁₋₁₈ alkyl and C₂₋₁₈ alkenyl,
n is 0 or 1, and
X⁻ is a pharmaceutically acceptable monovalent anion of inorganic acid or organic acid.

Even more specifically, in the above formula 1,
R₁ is a substituted or unsubstituted C₃₋₄ alkylene group,
each of R₂, R₃ and R₄ is independently a substituted or unsubstituted C₆₋₈ alkylene group,
each of R₅, R₆ and R₇ is independently a substituted or unsubstituted, saturated or unsaturated monovalent C₅₋₁₅ hydrocarbon group,
each of R₈ and R₉ is independently a substituted or unsubstituted C₁₋₂ alkyl group, or is independently -R₁₀-(L₄)ₙ-R₁₁,
each R₁₀ is independently a substituted or unsubstituted C₆₋₈ alkylene group,
each R₁₁ is independently a substituted or unsubstituted, saturated or unsaturated monovalent C₅₋₁₅ hydrocarbon group,
each of L₁, L₂, L₃ and L₄ is independently selected from the group consisting of -C(O)O-, -OC(O)-, -C(O)N(R')-, -N(R')C(O)-, -P(O)(OR')O-, -S-S-, and C₂₋₅ alkenylene, wherein each R' is independently selected from the group consisting of hydrogen atom and C₁₋₆ alkyl,
n is 0 or 1, and
X⁻ is F⁻, Cl⁻, Br⁻, I⁻, nitrate anion, benzoate anion, methanesulfonate anion, acetate anion (CH₃COO⁻) (i.e., AcO⁻), or trihaloacetate anion (CF₃COO⁻).

Even more specifically, the cationic lipid may be one having a structure selected from the following formulas A to Q:

| Formula | Structure |
|---|---|
| A | 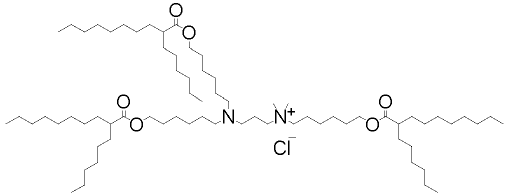 |
| B | 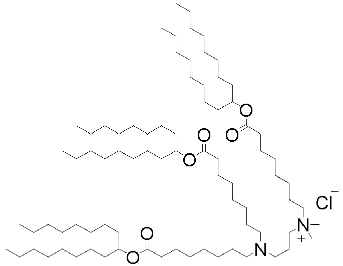 |
| C | 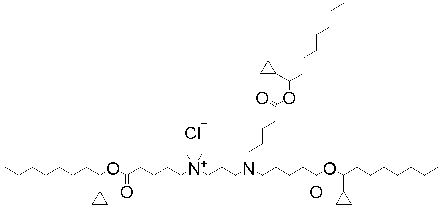 |
| D | 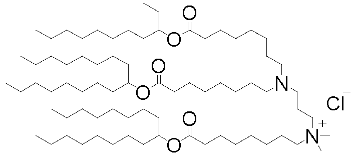 |
| E | 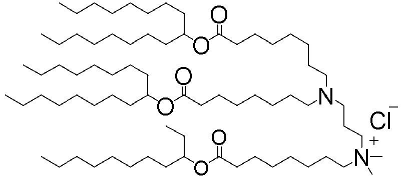 |
| F | 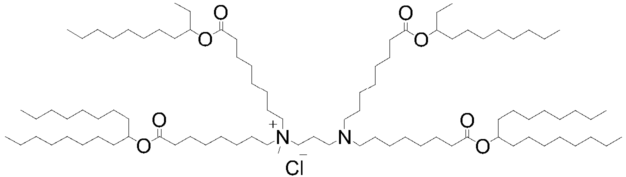 |
| G | 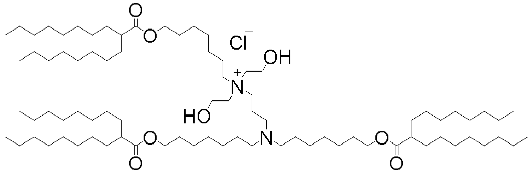 |
| H | 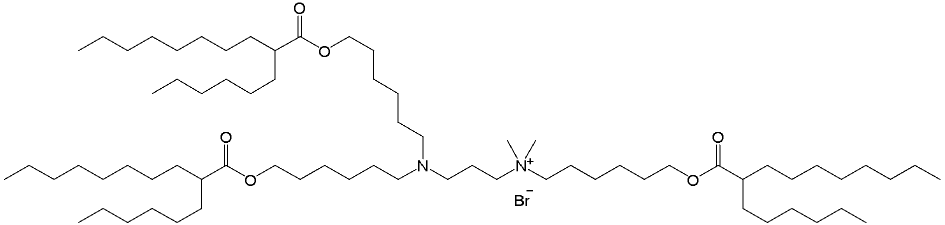 |
| I | 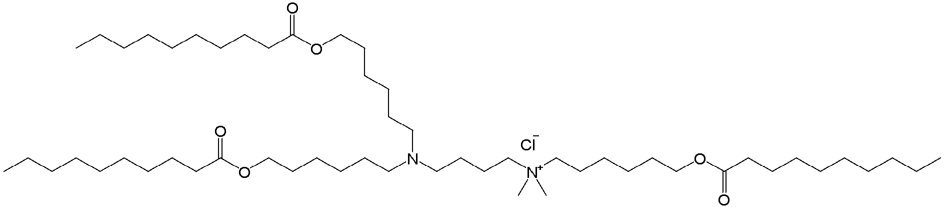 |
| J | 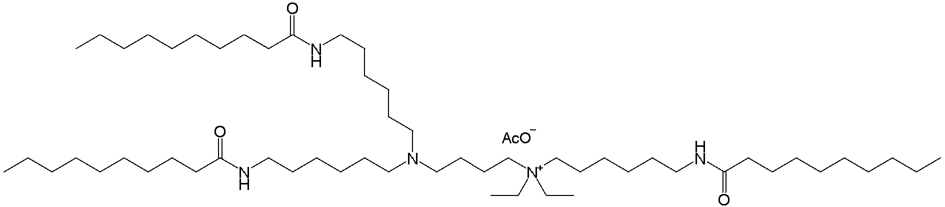 |
| K | 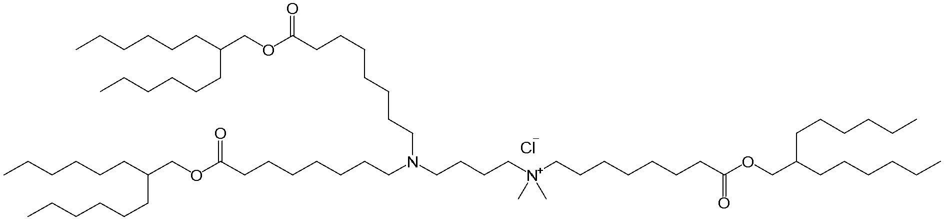 |
| L | 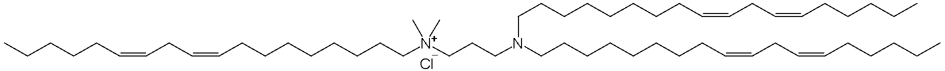 |
| M | 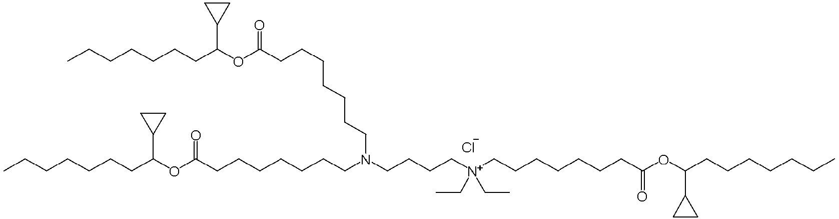 |
| N | 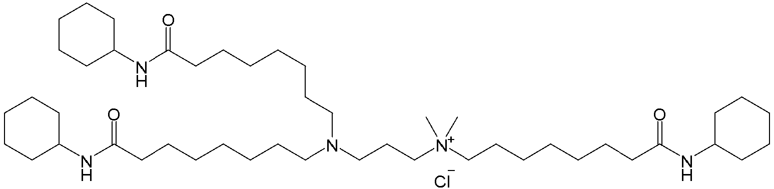 |
| O | 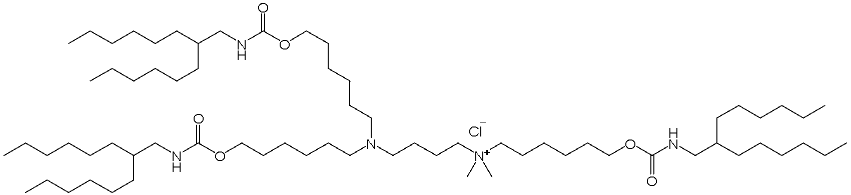 |
| P | 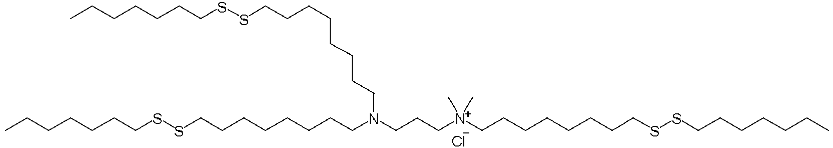 |
| Q | 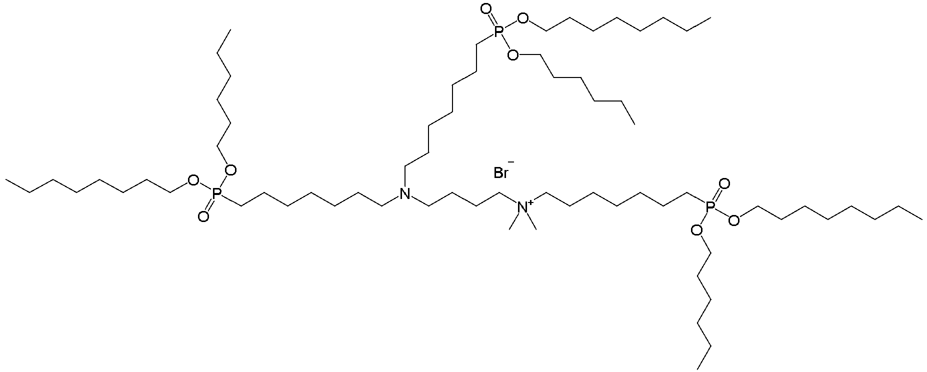 |

The second aspect of the present invention provides a method for preparing a cationic lipid having a structure represented by formula 1-1, comprising a step of reacting a compound of formula (a) with a compound of formula (b):

[Formula (a)] R₆-L₃-R₃-X

[Formula (b)] H₂N-R₁-N(-R₈)(-R₉)

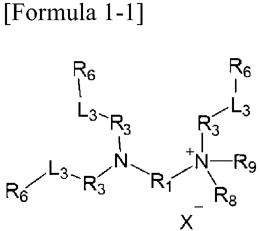

wherein,
R₁ is a substituted or unsubstituted alkylene group,
each R₃ is independently a substituted or unsubstituted alkylene group,
each R₆ is independently a substituted or unsubstituted, saturated or unsaturated monovalent hydrocarbon group,
each of R₈ and R₉ is independently a substituted or unsubstituted alkyl group or carbocyclic group,
each L₃ is independently selected from the group consisting of -C(O)O-, -OC(O)-, - OC(O)-L'-C(O)O-, -C(O)N(R')-, -N(R')C(O)-, -C(O)-, -C(S)-, -C(S)S-, -SC(S)-, -CH(OH)-, - P(O)(OR')O-, -S(O)₂-, -S-S-, alkenylene, arylene, and heteroarylene, wherein L' is a direct bond, alkylene or alkenylene, and each R' is independently selected from the group consisting of hydrogen atom, alkyl and alkenyl, and
X⁻ is a pharmaceutically acceptable monovalent anion.

The third aspect of the present invention provides a method for preparing a cationic lipid having a structure represented by formula 1-2 or formula 1-3, comprising steps of: (1) reacting a compound of formula (a) with a compound of formula (b) to obtain a compound of formula (c); and (2) reacting a compound of formula (c) with a compound of formula (d):

[Formula (a)] R₆-L₃-R₃-X

[Formula (b)] H₂N-R₁-N(-R₈)(-R₉)

[Formula (c)] (R₆-L₃-R₃-)NH-R₁-N⁺(-R₈)(-R₉)(-R₃-L₃-R₆)

[Formula (d)] R₇-L₂-R₂-X

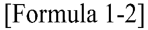

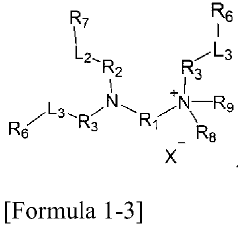

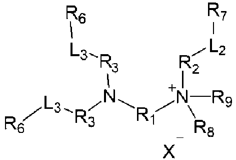

wherein,
R₁ is a substituted or unsubstituted alkylene group,
each of R₂ and R₃ is independently a substituted or unsubstituted alkylene group,
each of R₆ and R₇ is independently a substituted or unsubstituted, saturated or unsaturated monovalent hydrocarbon group,
each of R₈ and R₉ is independently a substituted or unsubstituted alkyl group or carbocyclic group,
each of L₂ and L₃ is independently selected from the group consisting of -C(O)O-, - OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, alkenylene, arylene, and heteroarylene, wherein L' is a direct bond, alkylene or alkenylene, and each R' is independently selected from the group consisting of hydrogen atom, alkyl and alkenyl, and
X⁻ is a pharmaceutically acceptable monovalent anion.

The fourth aspect of the present invention provides a method for preparing a cationic lipid having a structure represented by formula 1-4, comprising steps of: (1) reacting a compound of formula (a) with a compound of formula (b') to obtain a compound of formula (c'); (2) reacting a compound of formula (c') with a compound of formula (d) to obtain a compound of formula (e); and (3) reacting a compound of formula (e) with a compound of formula (f):

[Formula (a)] R₆-L₃-R₃-X

[Formula (b')] H₂N-R₁-NH₂

[Formula (c')] (R₆-L₃-R₃-)NH-R₁-NH(-R₃-L₃-R₆)

[Formula (d)] R₇-L₂-R₂-X

[Formula (e)] (R₆-L₃-R₃-)(R₇-L₂-R₂-)N-R₁-N(-R₂-L₂-R₇)(-R₃-L₃-R₆)

[Formula (f)] R₈-X

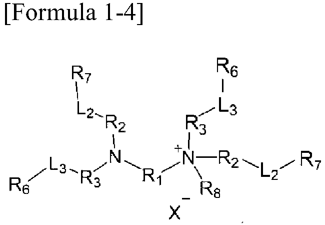

wherein,
R₁ is a substituted or unsubstituted alkylene group,
each of R₂ and R₃ is independently a substituted or unsubstituted alkylene group,
each of R₆ and R₇ is independently a substituted or unsubstituted, saturated or unsaturated monovalent hydrocarbon group,
each R₈ is independently a substituted or unsubstituted alkyl group or carbocyclic group,
each of L₂ and L₃ is independently selected from the group consisting of -C(O)O-, - OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, alkenylene, arylene, and heteroarylene, wherein L' is a direct bond, alkylene or alkenylene, and each R' is independently selected from the group consisting of hydrogen atom, alkyl and alkenyl, and
X⁻ is a pharmaceutically acceptable monovalent anion.

The fifth aspect of the present invention provides a method for preparing a cationic lipid having a structure represented by formula 1-5, comprising steps of: (1) reacting a compound of formula (a) with a compound of formula (b) to obtain a compound of formula (c"); and (2) reacting a compound of formula (c") with a compound of formula (d):

[Formula (a)] R₆-L₃-R₃-X

[Formula (b)] H₂N-R₁-N(-R₈)(-R₉)

[Formula (c")] (R₆-L₃-R₃-)NH-R₁-N(-R₈)(-R₉)

[Formula (d)] R₇-L₂-R₂-X

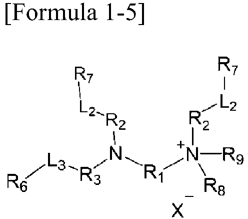

wherein,
R₁ is a substituted or unsubstituted alkylene group,
each of R₂ and R₃ is independently a substituted or unsubstituted alkylene group,
each of R₆ and R₇ is independently a substituted or unsubstituted, saturated or unsaturated monovalent hydrocarbon group,
each of R₈ and R₉ is independently a substituted or unsubstituted alkyl group or carbocyclic group,
each of L₂ and L₃ is independently selected from the group consisting of -C(O)O-, - OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, alkenylene, arylene, and heteroarylene, wherein L' is a direct bond, alkylene or alkenylene, and each R' is independently selected from the group consisting of hydrogen atom, alkyl and alkenyl, and
X⁻ is a pharmaceutically acceptable monovalent anion.

The sixth aspect of the present invention provides a composition for drug delivery comprising the cationic lipid according to the present invention.

### EFFECT OF THE INVENTION

The cationic lipid according to the present invention can easily form a complex with anionic drug due to its specific structure having quaternary amine and tertiary amine at the same time, and by utilizing the complex, the drug can be efficiently delivered into the targeted living tissue.

### BRIEF EXPLANATION OF THE DRAWINGS

Figure 1 is a reaction scheme for the synthesis procedure of Formula A compound conducted in Example 1.
Figure 2 is a reaction scheme for the synthesis procedure of Formula B compound conducted in Example 2.
Figure 3 is a reaction scheme for the synthesis procedure of Formula C compound conducted in Example 3.
Figure 4 is a reaction scheme for the synthesis procedures of Formulas D and E compounds conducted in Example 4.
Figure 5 is a reaction scheme for the synthesis procedure of Formula F compound conducted in Example 5.
Figure 6 is a reaction scheme for the synthesis procedure of Formula G compound conducted in Example 6.
Figure 7 is a reaction scheme for the synthesis procedure of Formula H compound conducted in Example 7.

### CONCRETE MODE FOR CARRYING OUT THE INVENTION

The present invention will be explained in detail below.

A cationic lipid provided by the first aspect of the present invention has a structure represented by the following formula 1: 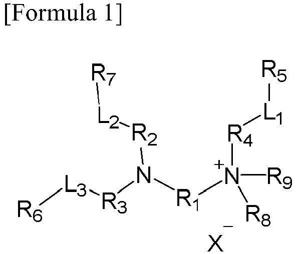 wherein, in the above formula 1,
R₁ is a substituted or unsubstituted alkylene group,
each of R₂, R₃ and R₄ is independently a substituted or unsubstituted alkylene group,
each of R₅, R₆ and R₇ is independently a substituted or unsubstituted, saturated or unsaturated monovalent hydrocarbon group,
each of R₈ and R₉ is independently a substituted or unsubstituted alkyl group or carbocyclic group, or is independently -R₁₀-(L₄)ₙ-R₁₁,
each R₁₀ is independently a substituted or unsubstituted alkylene group,
each R₁₁ is independently a substituted or unsubstituted, saturated or unsaturated monovalent hydrocarbon group,
each of L₁, L₂, L₃ and L₄ is independently selected from the group consisting of -C(O)O-, -OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, alkenylene, arylene, and heteroarylene, wherein L' is a direct bond, alkylene or alkenylene, and each R' is independently selected from the group consisting of hydrogen atom, alkyl and alkenyl,
n is 0 or 1, and
X⁻ is a pharmaceutically acceptable monovalent anion.

As used herein, the expression "substituted or unsubstituted" for any group means that, unless specified otherwise, the group is not substituted, or is substituted with one or more substituents selected from -OH, halogen atom, C₁₋₆ alkyl group, C₁₋₆ alkoxy group, C₁₋₆ halogenated alkyl group, C₁₋₆ halogenated alkoxy group, C₃₋₂₀ cycloalkyl group, C₃₋₂₀ heterocycloalkyl group, C₆₋₂₀ aryl group or C₃₋₂₀ heteroaryl group.

As used herein, the expression "hetero-" for any group (e.g., heteroaryl, heterocycloalkyl, etc.) means that, unless specified otherwise, the group has one or more (e.g., 1 to 3) heteroatoms selected from N, O and S.

As used herein, "monovalent hydrocarbon group" may be branched or not branched, cyclic or not cyclic, or aromatic.

According to an embodiment of the present invention, in the above formula 1,
R₁ may be a substituted or unsubstituted C₁₋₆ alkylene group,
each of R₂, R₃ and R₄ may be independently a substituted or unsubstituted C₃₋₁₂ alkylene group,
each of R₅, R₆ and R₇ may be independently a substituted or unsubstituted, saturated or unsaturated monovalent C₃₋₂₀ hydrocarbon group,
each of R₈ and R₉ may be independently a substituted or unsubstituted C₁₋₆ alkyl group or C₃₋₆ carbocyclic group, or may be independently -R₁₀-(L₄)ₙ-R₁₁,
each R₁₀ may be independently a substituted or unsubstituted C₃₋₁₂ alkylene group,
each R₁₁ may be independently a substituted or unsubstituted, saturated or unsaturated monovalent C₃₋₂₀ hydrocarbon group,
each of L₁, L₂, L₃ and L₄ may be independently selected from the group consisting of - C(O)O-, -OC(O)-, -OC(O)-L'-C(O)O-, -C(O)N(R')-, -N(R')C(O)-, -C(O)-, -C(S)-, -C(S)S-, - SC(S)-, -CH(OH)-, -P(O)(OR')O-, -S(O)₂-, -S-S-, C₂₋₆ alkenylene, C₆₋₂₀ arylene, and C₃₋₂₀ heteroarylene, wherein L' may be a direct bond, C₁₋₁₃ alkylene or C₂₋₁₃ alkenylene, and each R' may be independently selected from the group consisting of hydrogen atom, C₁₋₁₈ alkyl and C₂₋₁₈ alkenyl,
n is 0 or 1, and
X⁻ may be a pharmaceutically acceptable monovalent anion of inorganic acid or organic acid.

Even more specifically, in the above formula 1,
R₁ may be a substituted or unsubstituted C₃₋₄ alkylene group,
each of R₂, R₃ and R₄ may be independently a substituted or unsubstituted C₆₋₈ alkylene group,
each of R₅, R₆ and R₇ may be independently a substituted or unsubstituted, saturated or unsaturated monovalent C₅₋₁₅ hydrocarbon group,
each of R₈ and R₉ may be independently a substituted or unsubstituted C₁₋₂ alkyl group, or may be independently -R₁₀-(L₄)ₙ-R₁₁,
each R₁₀ may be independently a substituted or unsubstituted C₆₋₈ alkylene group,
each R₁₁ may be independently a substituted or unsubstituted, saturated or unsaturated monovalent C₅₋₁₅ hydrocarbon group,
each of L₁, L₂, L₃ and L₄ may be independently selected from the group consisting of - C(O)O-, -OC(O)-, -C(O)N(R')-, -N(R')C(O)-, -P(O)(OR')O-, -S-S-, and C₂₋₅ alkenylene, wherein each R' may be independently selected from the group consisting of hydrogen atom and C₁₋₆ alkyl,
n is 0 or 1, and
X⁻ may be F⁻, Cl⁻, Br⁻, I⁻, nitrate anion (NO₃⁻), benzoate anion (C₆H₅COO⁻), methanesulfonate anion, acetate anion (CH₃COO⁻) (i.e., AcO⁻), or trihaloacetate anion (CF₃COO⁻).

Even more specifically, the cationic lipid may be one having a structure selected from the following formulas A to Q:

| Formula | Structure |
|---|---|
| A | 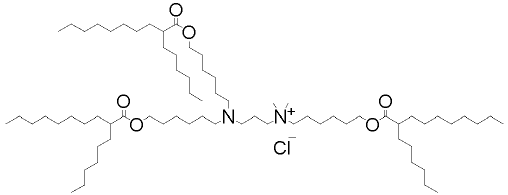 |
| B | 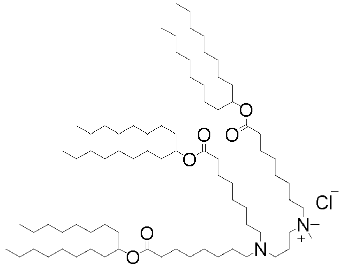 |
| C | 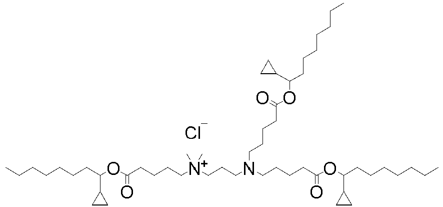 |
| D | 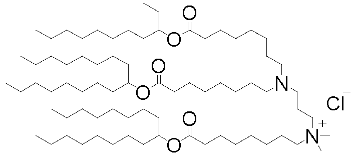 |
| E | 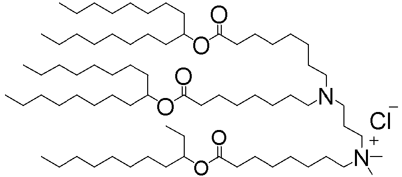 |
| F | 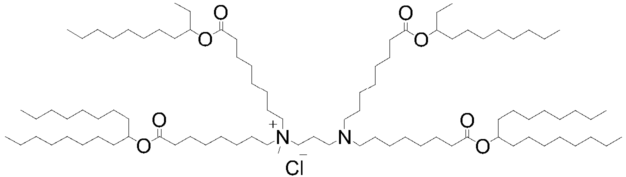 |
| G | 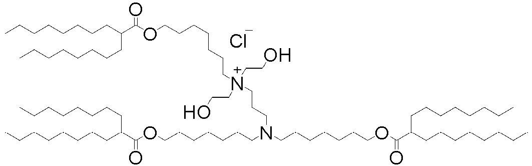 |
| H | 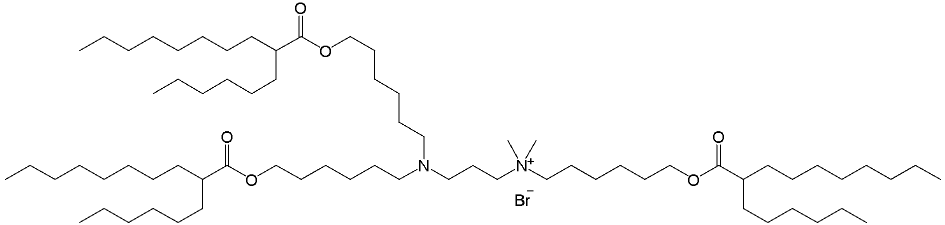 |
| I | 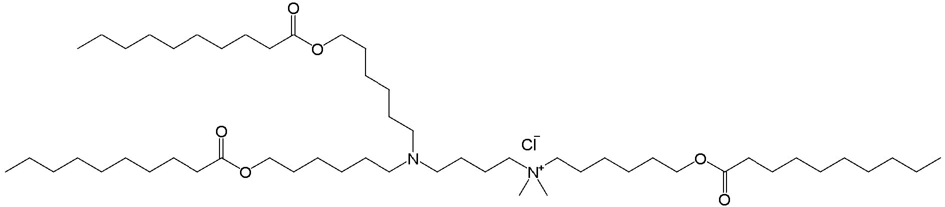 |
| J | 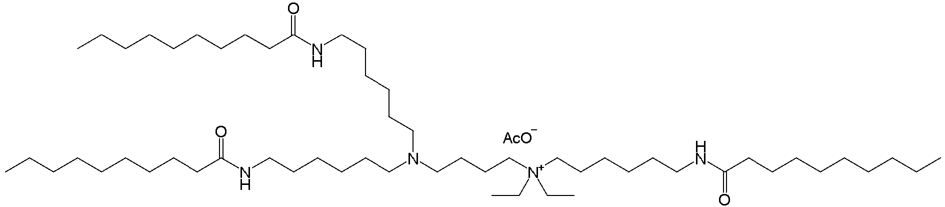 |
| K | 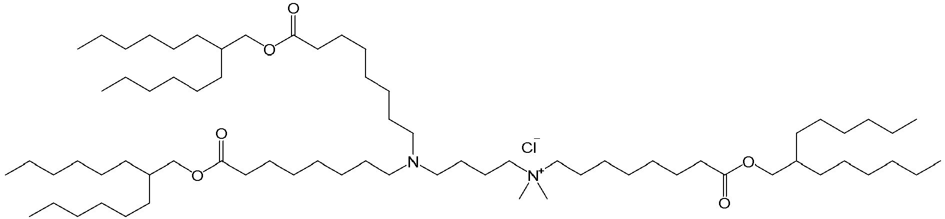 |
| L | 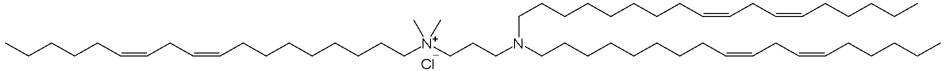 |
| M | 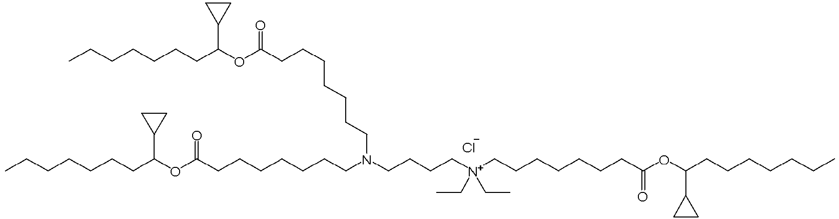 |
| N | 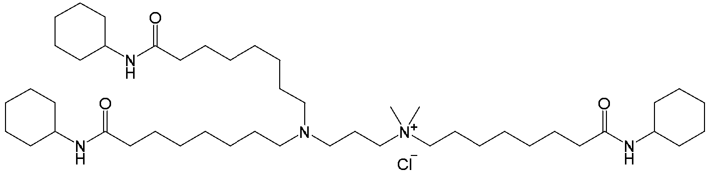 |
| O | 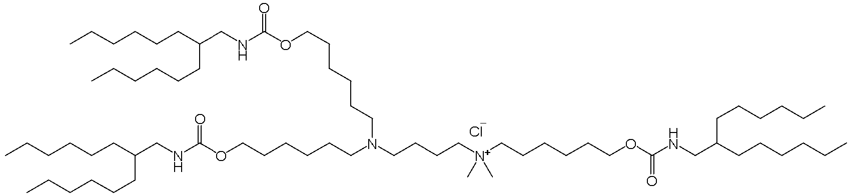 |
| P | 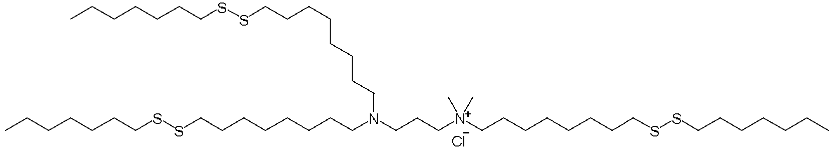 |
| Q | 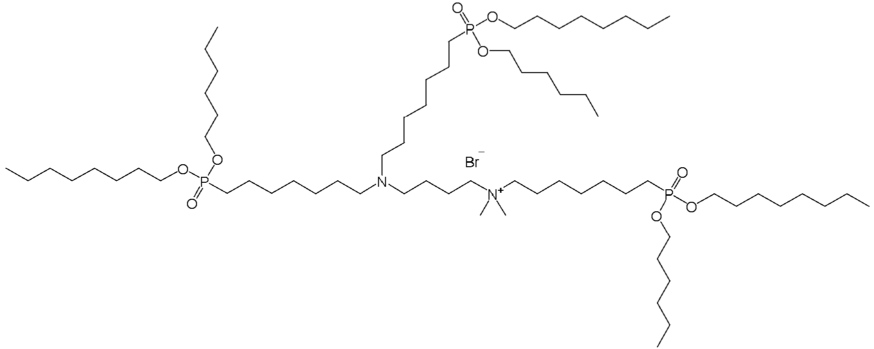 |

The second aspect of the present invention provides a method for preparing a cationic lipid having a structure represented by formula 1-1, comprising a step of reacting a compound of formula (a) with a compound of formula (b):

[Formula (a)] R₆-L₃-R₃-X

[Formula (b)] H₂N-R₁-N(-R₈)(-R₉)

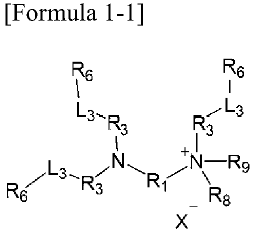

wherein,
R₁, R₃, R₆, R₈, R₉, L₃ and X⁻ are the same as defined above.

In an embodiment of the method for preparing a cationic lipid according to the second aspect of the present invention, the reaction between the compound of formula (a) and the compound of formula (b) may be carried out in a solvent (e.g., ethanol or acetonitrile (ACN), etc.) in the presence of a catalyst (e.g., Na₂CO₃, K₂CO₃, N,N-diisopropylethylamine (DIEA), etc.) at an elevated temperature (e.g., 80°C to 100°C), but it is not limited thereto.

The third aspect of the present invention provides a method for preparing a cationic lipid having a structure represented by formula 1-2 or formula 1-3, comprising steps of: (1) reacting a compound of formula (a) with a compound of formula (b) to obtain a compound of formula (c); and (2) reacting a compound of formula (c) with a compound of formula (d):

[Formula (a)] R₆-L₃-R₃-X

[Formula (b)] H₂N-R₁-N(-R₈)(-R₉)

[Formula (c)] (R₆-L₃-R₃-)NH-R₁-N⁺(-R₈)(-R₉)(-R₃-L₃-R₆)

[Formula (d)] R₇-L₂-R₂-X

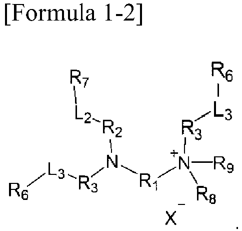

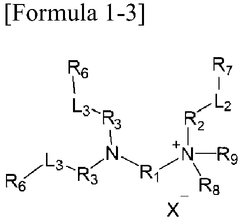

wherein,
R₁ to R₃, R₆ to R₉, L₂, L₃ and X⁻ are the same as defined above.

In an embodiment of the method for preparing a cationic lipid according to the third aspect of the present invention, the reaction between the compound of formula (a) and the compound of formula (b) may be carried out in a solvent (e.g., ethanol or acetonitrile (ACN), etc.) in the presence of a catalyst (e.g., Na₂CO₃, K₂CO₃, N,N-diisopropylethylamine (DIEA), etc.) at an elevated temperature (e.g., 80°C to 100°C), but it is not limited thereto. In addition, the reaction between the compound of formula (c) and the compound of formula (d) may be carried out in a solvent (e.g., ethanol or acetonitrile (ACN), etc.) in the presence of a catalyst (e.g., Na₂CO₃, K₂CO₃, N,N-diisopropylethylamine (DIEA), etc.) at an elevated temperature (e.g., 80°C to 100°C), but it is not limited thereto.

The fourth aspect of the present invention provides a method for preparing a cationic lipid having a structure represented by formula 1-4, comprising steps of: (1) reacting a compound of formula (a) with a compound of formula (b') to obtain a compound of formula (c'); (2) reacting a compound of formula (c') with a compound of formula (d) to obtain a compound of formula (e); and (3) reacting a compound of formula (e) with a compound of formula (f):

[Formula (a)] R₆-L₃-R₃-X

[Formula (b')] H₂N-R₁-NH₂

[Formula (c')] (R₆-L₃-R₃-)NH-R₁-NH(-R₃-L₃-R₆)

[Formula (d)] R₇-L₂-R₂-X

[Formula (e)] (R₆-L₃-R₃-)(R₇-L₂-R₂-)N-R₁-N(-R₂-L₂-R₇)(-R₃-L₃-R₆)

[Formula (f)] R₈-X

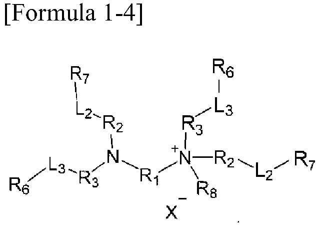

wherein,
R₁ to R₃, R₆ to R₈, L₂, L₃ and X⁻ are the same as defined above.

In an embodiment of the method for preparing a cationic lipid according to the fourth aspect of the present invention, the reaction between the compound of formula (a) and the compound of formula (b') may be carried out in a solvent (e.g., ethanol or acetonitrile (ACN), etc.) in the presence of a catalyst (e.g., Na₂CO₃, K₂CO₃, etc.) at an elevated temperature (e.g., 40°C to 60°C), but it is not limited thereto. In addition, the reaction between the compound of formula (c') and the compound of formula (d) may be carried out in a solvent (e.g., ethanol or acetonitrile (ACN), etc.) in the presence of a catalyst (e.g., Na₂CO₃, K₂CO₃, etc.) at an elevated temperature (e.g., 40°C to 60°C), but it is not limited thereto. Furthermore, the reaction between the compound of formula (e) and the compound of formula (f) may be carried out in a solvent (e.g., ethanol or acetonitrile (ACN), etc.) in the presence of a catalyst (e.g., Na₂CO₃, K₂CO₃, etc.), and for example, at a temperature of 20°C to 40°C, but it is not limited thereto.

The fifth aspect of the present invention provides a method for preparing a cationic lipid having a structure represented by formula 1-5, comprising steps of: (1) reacting a compound of formula (a) with a compound of formula (b) to obtain a compound of formula (c"); and (2) reacting a compound of formula (c") with a compound of formula (d):

[Formula (a)] R₆-L₃-R₃-X

[Formula (b)] H₂N-R₁-N(-R₈)(-R₉)

[Formula (c")] (R₆-L₃-R₃-)NH-R₁-N(-R₈)(-R₉)

[Formula (d)] R₇-L₂-R₂-X

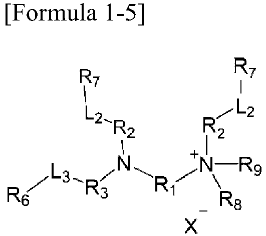

wherein,
R₁ to R₃, R₆ to R₉, L₂, L₃ and X⁻ are the same as defined above.

In an embodiment of the method for preparing a cationic lipid according to the fifth aspect of the present invention, the reaction between the compound of formula (a) and the compound of formula (b) may be carried out in a solvent (e.g., ethanol or acetonitrile (ACN), etc.) in the presence of a catalyst (e.g., Na₂CO₃, K₂CO₃, N,N-diisopropylethylamine (DIEA), etc.) at an elevated temperature (e.g., 80°C to 100°C), but it is not limited thereto. In addition, the reaction between the compound of formula (c") and the compound of formula (d) may be carried out in a solvent (e.g., ethanol or acetonitrile (ACN), etc.) in the presence of a catalyst (e.g., Na₂CO₃, K₂CO₃, N,N-diisopropylethylamine (DIEA), etc.) at an elevated temperature (e.g., 80°C to 100°C), but it is not limited thereto.

The cationic lipid according to the present invention comprises quaternary amine and tertiary amine together and thereby can easily form a complex with anionic drug, and thus is useful for drug delivery.

Thus, the sixth aspect of the present invention provides a composition for drug delivery comprising the cationic lipid according to the present invention.

In an embodiment, the drug may be selected from nucleic acid, polypeptide, virus or combination thereof.

The "nucleic acid" may be, for example, DNA, RNA, siRNA, shRNA, miRNA, mRNA, aptamer, antisense oligonucleotide, or a combination thereof, but it is not limited thereto.

The "polypeptide" may mean a protein having activity in the body such as antibody or fragment thereof, cytokine, hormone or analog thereof, or a protein that can be recognized as antigen through a series of processes in the body, including polypeptide sequence of antigen, analog or precursor thereof.

In an embodiment, the lipid of the present invention may form a complex with the drug, and the complex is encapsulated within nanoparticle structure formed by an amphiphilic block copolymer.

In an embodiment, the amphiphilic block copolymer may be an A-B type block copolymer comprising a hydrophilic A block and a hydrophobic B block. In an aqueous environment, the A-B type block copolymer forms core-shell type polymer nanoparticle wherein the hydrophobic B block forms the core (inner wall) and the hydrophilic A block forms the shell (outer wall).

In an embodiment, the hydrophilic A block may be one or more selected from the group consisting of polyalkylene glycol, polyvinyl alcohol, polyvinyl pyrrolidone, polyacrylamide, and derivatives thereof.

More concretely, the hydrophilic A block may be one or more selected from the group consisting of monomethoxypolyethylene glycol (mPEG), monoacetoxypolyethylene glycol, polyethylene glycol, copolymer of polyethylene and propylene glycol, and polyvinylpyrrolidone.

Also, if necessary, the end of the hydrophilic A block may be chemically combined with a functional group or ligand capable of reaching specific tissue or cell, or a functional group capable of promoting intracellular delivery, in order to control *in vivo* distribution of polymer nanoparticle carrier or to increase the efficiency of delivering the nanoparticle carrier into cell. In an embodiment, the functional group or ligand may be one or more selected from the group consisting of monosaccharides, polysaccharides, vitamins, peptides, proteins, and antibodies to cell surface receptors. More concretely, the functional group or ligand may be one or more selected from the group consisting of anisamide, vitamin B9 (folic acid), vitamin B12, vitamin A, galactose, lactose, mannose, hyaluronic acid, RGD peptide, NGR peptide, transferrin, antibody to transferrin receptor, etc.

The hydrophobic B block is a biocompatible, biodegradable polymer, and in an embodiment, it may be one or more selected from the group consisting of polyester, polyanhydride, polyamino acid, polyorthoester and polyphosphazine.

More concretely, the hydrophobic B block may be one or more selected from the group consisting of polylactide (PLA), polyglycolide, polycaprolactone, polydioxan-2-one, copolymer of polylactide and glycolide, copolymer of polylactide and polydioxan-2-one, copolymer of polylactide and and polycaprolactone, and a copolymer of polyglycolide and polycaprolactone.

Also, in an embodiment, in order to increase the hydrophobicity of the hydrophobic B block and thereby improve the stability of the nanoparticle, the hydrophobic B block may be modified by chemically combining the hydroxyl group at the end of the hydrophobic B block with tocopherol, cholesterol, or fatty acid having 10 to 24 carbons.

The present invention will be explained below in more detail with reference to the following Examples. However, the Examples are only to illustrate the invention, and the scope of the present invention is not limited thereby in any manner.

### EXAMPLES

### Example 1

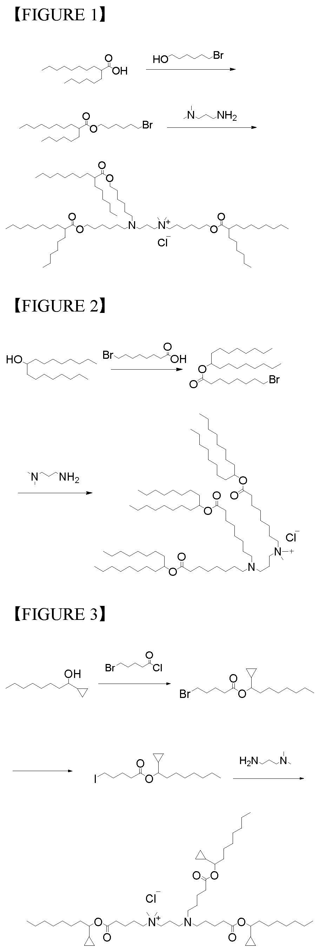

The compound of the following formula A was prepared according to the synthesis scheme shown in Figure 1.

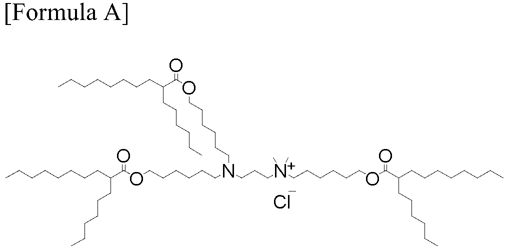

### (1) Synthesis of 6-bromohexyl 2-hexyldecanoate

In a 2000 mL 3-neck round-bottom flask (RBF), 2-hexyldecanoic acid (100 g, 390 mmol, 1.00 eq), 6-bromohexan-1-ol (91.8 g, 507 mmol, 1.30 eq) and toluene (1000 mL) were placed, and H₂SO₄ (7.65 g, 78.0 mmol, 0.20 eq) was added to the mixture. The mixture was degassed and purged with N₂ gas, and then stirred at 120°C for 16 hours. After cooling to room temperature, the mixture was concentrated under reduced pressure to remove the solvent. The residue was purified by silica column chromatography (petroleum ether:ethyl acetate (EtOAc)=10:1→1:100) to obtain 6-bromohexyl 2-hexyl decanoate (135 g, 322 mmol, 82.5% yield) in a form of pale yellow oil.

### (2) Synthesis of the compound of formula A

In a 500 mL 3-neck RBF, 6-bromohexyl 2-hexyl decanoate (15.0 g, 35.8 mmol, 3.50 eq), N¹,N¹-dimethylpropane-1,3-diamine and ethanol (EtOH) (150 mL) were placed, and Na₂CO₃ (3.25 g, 30.7 mmol, 3.00 eq) was added to the mixture. After purging three times with N₂ gas, the mixture was stirred at 90°C for 48 hours. After cooling to room temperature, the solvent was removed under reduced pressure. The residue was extracted with a saturated aqueous solution of Na₂CO₃ and dichloromethane (DCM), and after taking the DCM layer, the solvent was removed under reduced pressure to obtain residue A. Residue A was dissolved in EtOH (60 mL), and H₂O (60 mL) and an excess amount of Na₂CO₃ were added thereto, and the mixture was stirred at room temperature for 16 hours. After filtering the mixture, the EtOH was removed from the filtrate under reduced pressure. The residual solution was extracted with DCM, and the DCM layer was taken, and the solvent was removed under reduced pressure to obtain residue B. Residue B was purified by reversed-phase HPLC (column: C1 250×80 mm, 10 µm; mobile phase: [HCl aqueous solution-ACN]; gradient: 55%-85% for 20 minutes) to obtain the compound of formula A (12.0 g, 10.7 mmol, 35.5% yield) in a form of yellow solid.

¹H NMR (400 MHz, CHLOROFORM-*d*): *δ* 4.07-4.04 (m, 4H), 3.45 (td, 2H), 3.28 (s, 6H), 3.08 (br, 4H), 2.72 (br, H), 2.33-2.27 (m, 3H), 1.84 (br, 6H), 1.68-1.45 (m, 20H), 1.45-1.42 (m, 18H), 1.32-1.25 (m, 62H), 0.89 (t, 18H)

### Example 2

The compound of the following formula B was prepared according to the synthesis scheme shown in Figure 2.

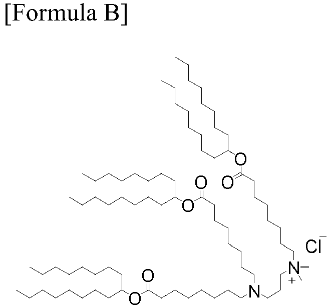

### (1) Synthesis of heptadecan-9-yl 8-bromooctanoate

In a 1000 mL of 3-neck RBF, heptadecan-9-ol (43.00 g, 168.00 mmol, 1.00 eq), 8-bromooctanic acid (44.90 g, 201.00 mmol, 1.20 eq), 4-dimethylaminopyridine (DMAP) (20.50 g, 168.00 mmol, 1.00 eq), 1-ethyl-3-(3-dimethylaminopropyl)carbodiimide (EDCI) (48.20 g, 252.00 mmol, 1.50 eq) and triethylamine (TEA) (17.00 g, 168.00 mmol, 23.30 mL, 1.00 eq) were placed, and DCM (430 mL) was added thereto. After purging with N₂ three times, the mixture was stirred under N₂ at 25°C for 16 hours. 500 mL of water was added to the reaction mixture at 20°C, and after quenching, the mixture was extracted with 900 mL of DCM (300 mL * 3). The collected organic layer was concentrated under reduced pressure, and the obtained residue was then purified by silica column chromatography (petroleum ether:EtOAc = 100:1→1:1) to obtain heptadecan-9-yl 8-bromooctanoate (36.00 g, 78.00 mmol, 46.5% yield) in a form of pale yellow oil.

### (2) Synthesis of the compound of formula B

In a 100 mL of 3-neck RBF, heptadecan-9-yl 8-bromooctanoate (3.16 g, 6.85 mmol, 3.50 eq), N¹,N¹-dimethylpropane-1,3-diamine (0.20 g, 1.96 mmol, 1.00 eq), and N,N-diisopropylethylamine (DIEA) (1.26 g, 9.79 mmol, 1.70 mL, 5.00 eq) were placed, and EtOH (5 mL) was added thereto. After purging with N₂ three times, the mixture was stirred for 16 hours under N₂ condition at 95°C. The reaction mixture was filtered and concentrated under reduced pressure to remove the solvent. The mixture was extracted with saturated Na₂CO₃ and 300 mL (100 mL x 3) of DCM, and the collected organic layer was concentrated under reduced pressure to obtain residue A. Residue A was dissolved in EtOH (50 mL), then 60 mL of water and an excess amount of Na₂CO₃ were added to the mixture, and the mixture was stirred at 25°C for 16 hours. The mixture was filtered and concentrated under reduced pressure to remove EtOH, and then the aqueous layer was extracted with 300 mL (100 mL x 3) of DCM. The collected organic layer was concentrated under reduced pressure to obtain residue B. Residue B was purified by prep-HPLC (column: C1 250×80 mm, 10 µm; mobile phase: [HCl aqueous solution-ACN]; gradient: 55%-85% for 20 minutes) and concentrated under reduced pressure to remove the solvent, thereby to obtain the compound of formula B (1.52 g, 1.19 mmol, 60.6% yield) in a form of pale yellow syrup.

¹H NMR (400 MHz, CHLOROFORM-*d*): δ 4.86 (t, *J*=6.2 Hz, 3H), 4.15 (br d, *J=*1.3 Hz, 2H), 3.44 - 3.32 (m, 4H), 3.28 (s, 6H), 3.14 - 2.94 (m, 4H), 2.72 - 2.58 (m, 2H), 2.28 (dt, *J*=2.1, 7.4 Hz, 6H), 1.96 - 1.74 (m, 12H), 1.65 - 1.58 (m, 6H), 1.51 (br d, *J*=5.8 Hz, 12H), 1.41 - 1.25 (m, 84H), 0.94 - 0.83 (m, 18H)

### Example 3

The compound of the following formula C was prepared according to the synthesis scheme shown in Figure 3.

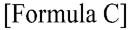

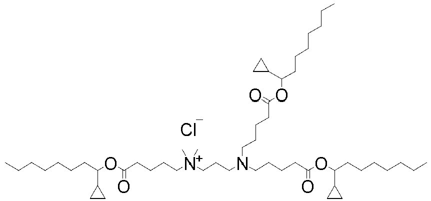

### (1) Synthesis of 1-cyclopropyloctyl 5-bromopentanoate

In a 500 mL of 3-neck RBF, 1-cyclopropyloctan-1-ol (15.00 g, 88.90 mmol, 1.00 eq), DMAP (2.15 g, 17.60 mmol, 0.20 eq) and TEA (17.80 g, 176.00 mmol, 24.50 mL, 2.00 eq) were placed, and DCM (150 mL) was added thereto. After purging the mixture with N₂ three times, 5-bromopentanoyl chloride (26.40 g, 132.00 mmol, 17.70 mL, 1.50 eq) was added to the solution at 0°C. After stirring the solution at 25°C for 16 hours, 100 mL of H₂O was added to the reaction mixture at 25°C for quenching. The mixture was extracted with 1500 mL of DCM (500 mL * 3), dried with Na₂SO₄, filtered, and concentrated under reduced pressure to obtain the residue. The residue was purified by silica column chromatography (petroleum ether:EtOAc=10:1) to obtain 1-cyclopropyloctyl 5-bromopentanoate (20.0 g, 60.0 mmol, 68.1% yield) in a form of colorless oil.

### (2) Synthesis of 1-cyclopropyloctyl 5-iodopentanoate

In a 500 mL 3-neck RBF, 1-cyclopropyloctyl 5-bromopentanoate (20.00 g, 60.00 mmol, 1.00 eq) and KI (19.90 g, 120.00 mmol, 2.00 eq) were placed, and acetonitrile (ACN) (200 mL) was added thereto. The mixture was purged with N₂ three times and stirred for 16 hours under N₂ condition at 90°C. The mixture was filtered and concentrated under reduced pressure to obtain 1-cyclopropyloctyl 5-iodopentanoate (18.00 g, 47.30 mmol, 78.9% yield) in a form of yellow oil.

### (3) Synthesis of the compound of formula C

In a a 50 mL 3-neck RBF, N¹,N¹-dimethylpropane-1,3-diamine (0.20 g, 1.96 mmol, 0.33 eq), 1-cyclopropyloctyl 5-iodopentanoate (2.23 g, 5.87 mmol, 1.00 eq) and K₂CO₃ (812.00 mg, 5.87 mmol, 3.00 eq) were placed, and EtOH (2 mL) and ACN (2 mL) were added thereto. The mixture was purged with N₂ three times and stirred for 12 hours under N₂ condition at 80°C. The mixture was filtered and concentrated under reduced pressure to obtain the residue. The residue was purified by prep-HPLC (column: C1 250×80 mm, 10 µm; mobile phase: [HCl aqueous solution-ACN]; gradient: 55%-85% for 20 minutes) to obtain the compound of formula C (0.19 g, 221.00 µmol, 11.3% yield) in a form of yellow syrup.

¹H NMR (400 MHz, CHLOROFORM-*d*): *δ* 0.26 (dt, *J*=9.57, 4.85 Hz, 3 H), 0.34 (dq, J=9.54, 4.87 Hz, 3 H), 0.40 - 0.49 (m, 3 H), 0.51 - 0.60 (m, 3 H), 0.83 - 0.92 (m, 9 H), 0.92 - 1.01 (m, 3 H), 1.27 (br s, 30 H), 1.39 - 1.47 (m, 4 H), 1.55 - 1.69 (m, 10 H), 1.70 - 1.78 (m, 2 H), 1.78 - 1.92 (m, 4 H), 2.32 (t, *J*=7.00 Hz, 4 H), 2.36 - 2.46 (m, 6 H), 2.50 (br t, *J*=5.88 Hz, 2 H), 3.38 (s, 6 H), 3.48 - 3.60 (m, 2 H), 3.60 - 3.74 (m, 2 H), 4.14 - 4.33 (m, 3 H)

### Example 4

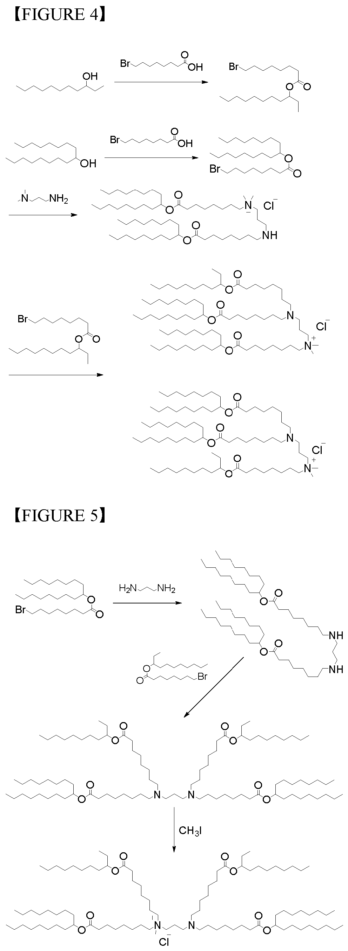

The compounds of the following formulas D and E were prepared according to the synthesis scheme shown in Figure 4.

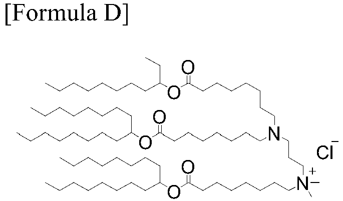

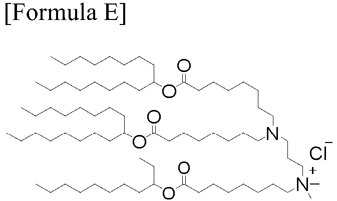

### (1) Synthesis of undecan-3-yl 8-bromooctanoate

In a 500 mL 3-neck RBF, undecane-3-ol (25.00 g, 145.00 mmol, 1.00 eq) dissolved in toluene (250 mL) was placed, and 8-bromooctanic acid (48.60 g, 218.00 mmol, 1.50 eq) was added thereto. Then, H₂SO₄ (2.13 g, 21.8 mmol, 0.15 eq) was added. The mixture was purged with N₂ three times and stirred at 120°C for 16 hours. The mixture was concentrated under reduced pressure to obtain the residue. The residue was purified by silica column chromatography (petroleum ether:EtOAc=10:1→1:100) to obtain undecane-3-yl 8-bromooctanoate (40.00 g, 106.00 mmol, 73.1% yield) in a form of yellow oil.

### (2) Synthesis of heptadecan-9-yl 8-bromooctanoate

In a 5000 mL 3-neck RBF, heptadecan-9-ol (235.00 g, 916.00 mmol, 1.00 eq) dissolved in toluene (2.35 L) was placed, and 8-bromooctanic acid (245.00 g, 1.10 mol, 1.20 eq) was added thereto. Then, H₂SO₄ (18.00 g, 183.00 mmol, 0.20 eq) was added. The mixture was purged with N₂ three times and stirred at 120°C for 16 hours. The mixture was concentrated under reduced pressure to obtain the residue. The residue was purified by silica column chromatography (petroleum ether:EtOAc=10:1→1:100) to obtain heptadecan-9-yl 8-bromooctanoate (470.00 g, 1.02 mol, 55.6% yield) in a form of yellow oil.

### (3) Synthesis of 8-(heptadecan-9-yloxy)-N-(3-((8-(heptadecan-9-yloxy)-8-oxooctyl)amino)propyl)-N,N-dimethyl-8-oxooctan-1-aminium chloride

In a 250 mL 3-neck RBF, heptadecan-9-yl 8-bromooctanoate (10.00 g, 21.70 mmol, 2.20 eq), N¹,N¹-dimethylpropane-1,3-diamine (1.01 g, 9.85 mmol, 1.00 eq), Na₂CO₃ (2.09 g, 19.70 mmol, 2.00 eq) and DIEA (3.82 g, 29.50 mmol, 3.00 eq) dissolved in EtOH (100 mL) were placed. After stirring the mixture at 90°C for 16 hours, the reaction mixture was concentrated under reduced pressure. The residue was purified by silica column chromatography (DCM:MeOH=10:1→1:100), and 6 g of the purified product was further purified by reverse-phase HPLC to obtain 8-(heptadecan-9-yloxy)-N-(3-((8-(heptadecan-9-yloxy)-8-oxo-octyl)amino)propyl)-N,N-dimethyl-8-oxo-octane-1-amine chloride (2.00 g, 2.31 mmol, 23.5% yield) in a form of yellow solid.

### (4) Synthesis of the compounds of formulas D and E

In a 50 mL 3-neck RBF, undecane-3-yl 8-bromooctanoate (314.00 mg, 833.00 µmol, 1.20 eq), 8-(heptadecan-9-yloxy)-N-(3-((8-(heptadecan-9-yloxy)-8-oxo-octyl)amino)propyl)-N,N-dimethyl-8-oxo-octane-1-aminium chloride (0.60 g, 694.00 µmol, 1.00 eq) and K₂CO₃ (115.00 mg, 833.00 µmol, 1.20 eq) dissolved in ACN (6 mL) were placed. The mixture was stirred at 90°C for 24 hours, and then the reaction mixture was concentrated under reduced pressure. The residue was purified by purification using reverse-phase HPLC (column: C1 250×80 mm, 10 µm; mobile phase: [HCl aqueous solution-ACN]; gradient: 45%-75% for 25 minutes) to obtain the compound of formula D (0.20 g, 167.00 µmol, 24.1% yield) in a form of yellow oil and the compound of formula E (0.30 g, 258.00 µmol, 37.2% yield) in a form of white solid.

### [Compound of formula D]

¹H NMR (400 MHz, CHLOROFORM-*d*): *δ* 11.60 (br s, 1H), 4.92 - 4.75 (m, 3H), 4.16 (br s, 2H), 3.48 - 3.33 (m, 4H), 3.29 (s, 6H), 3.14 - 2.96 (m, 4H), 2.66 (br s, 2H), 2.28 (tt, *J*=2.3, 7.3 Hz, 6H), 2.21 (br s, 2H), 1.82 (br s, 6H), 1.65 - 1.58 (m, 6H), 1.57 - 1.46 (m, 12H), 1.42 - 1.34 (m, 16H), 1.26 (br s, 54H), 0.88 (t, *J*=6.6 Hz, 18H)

### [Compound of formula E]

¹H NMR (400 MHz, CHLOROFORM-*d*): *δ* 11.60 (br s, 1H), 4.90 - 4.76 (m, 3H), 4.14 (br d, *J*=7.1 Hz, 2H), 3.48 - 3.32 (m, 4H), 3.29 (s, 6H), 3.14 - 2.97 (m, 4H), 2.65 (br s, 2H), 2.32 - 2.25 (m, 8H), 1.82 (br s, 6H), 1.65 - 1.58 (m, 6H), 1.57 - 1.46 (m, 12H), 1.43 - 1.34 (m, 16H), 1.32 - 1.24 (m, 59H), 0.93 - 0.82 (m, 18H)

### Example 5

The compound of the following formula F was prepared according to the synthesis scheme shown in Figure 5.

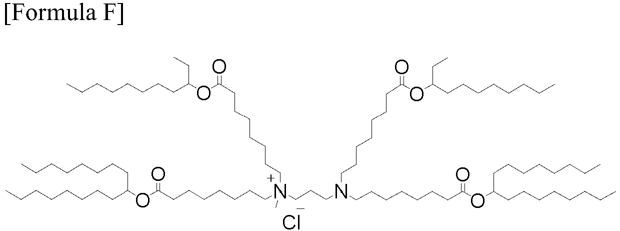

### (1) Synthesis of di(heptadecan-9-yl) 8,8'-(propane-1,3-diylbis(azanediyl))dioctanoate

In a 100 mL 3-neck RBF, heptadecan-9-yl 8-bromooctanoate (2.00 g, 4.33 mmol, 2.00 eq), propane-1,3-diamine (160.00 mg, 2.17 mmol, 1.00 eq) and K₂CO₃ (598.00 mg, 4.33 mmol, 2.00 eq) were placed, and ACN (20 mL) was added thereto. After purging the mixture with N₂ three times, the solution was stirred at 45°C for 16 hours. The reaction mixture was filtered and concentrated under reduced pressure to obtain the residue. The residue was purified by silica column chromatography (DCM:MeOH=10:1) to obtain di(heptadecan-9-yl) 8,8'-(propane-1,3-diylbis(azandyl))dioctanoate (0.80 g, 0.96 mmol, 11.0% yield) in a form of white solid.

### (2) Synthesis of di(heptadecan-9-yl) 8,8'-(9,33-diethyl-11,31-dioxo-10,32-dioxa-19,23-diazahentetracontane-19,23-diyl)dioctanoate

In a 50 mL 3-neck RBF, di(heptadecan-9-yl) 8,8'-(propane-1,3-diylbis(azandyl))dioctanoate (0.80 g, 0.96 mmol, 1.00 eq), undecane-3-yl 8-bromooctanoate (722.00 mg, 1.92 mmol, 1.00 eq) and K₂CO₃ (264.00 mg, 1.92 mmol, 2.00 eq) were placed, and ACN (8 mL) was added thereto. The solution was stirred at 55°C for 16 hours. The reaction mixture was filtered and concentrated under reduced pressure to obtain the residue. The residue was purified by prep-HPLC (water(HCl)-ACN) and concentrated under reduced pressure to remove ACN. An aqueous solution of NaHCO₃ (10 mL) was added to the aqueous solution from which ACN had been removed, and the mixture was neutralized overnight. Thereafter, the mixture was extracted with DCM (5 mL * 2), dried with Na₂SO₄, filtered, and subjected to reduced pressure to obtain di(heptadecan-9-yl) 8,8'-(9,33-diethyl-11,31-dioxo-10,32-dioxa-19,23-diazahentetracontan-19,23-diyl)dioctanoate (0.40 g, 0.28 mmol, 29.2% yield) in a form of yellow oil.

### (3) Synthesis of the compound of formula F

In a 50 mL 3-neck RBF, di(heptadecan-9-yl) 8,8'-(9,33-diethyl-11,31-dioxo-10,32-dioxa-19,23-diazahentetracontan-19,23-diyl)dioctanoate (0.40 g, 0.28 mmol, 1.00 eq) dissolved in THF (4 mL) and K₂CO₃ (42.50 mg, 0.31 mmol, 1.10 eq) were placed, and CH₃I (39.70 mg, 0.28 mmol, 1.00 eq) was added thereto. The solution was stirred at 25°C for 16 hours. 2M HCl (10 mL) was added to the reaction mixture for quenching, and distilled water (DW) (5 mL) was added for dilution, followed by extraction with EtOAc (5 mL * 2). The collected organic layer was concentrated under reduced pressure to obtain the residue. The residue was purified by prep-HPLC (Column: C1 250×80 mm, 10 µm; Mobile phase: [HCl aqueous solution-ACN]; Gradient: 45%-75% for 25 minutes), and concentrated under reduced pressure to remove ACN. NaHCO₃ aqueous solution (10 mL) was added to the aqueous solution from which ACN had been removed, and the mixture was neutralized overnight. Subsequently, the mixture was extracted with DCM (5 mL * 2), dried with Na₂SO₄, filtered, and subjected to reduced pressure to obtain the compound of formula F (0.40 g, 0.28 mmol, 29.2% yield) in a form of yellow oil.

¹HNMR: (400 MHz, CHLOROFORM-*d*) *δ* 0.74 - 1.00 (m, 24 H) 1.18 - 1.44 (m, 98 H) 1.47 - 1.56 (m, 14 H) 1.57 (br s, 8 H) 1.64 - 1.88 (m, 10 H) 2.18 - 2.38 (m, 9 H) 2.65 (ddd, *J=*10.98, 5.16, 1.88 Hz, 1 H) 2.90 - 3.15 (m, 3 H) 3.20 - 3.48 (m, 8 H) 3.92 - 4.12 (m, 2 H) 4.84 (dt, *J*=19.35, 6.14 Hz, 4 H)

### Example 6

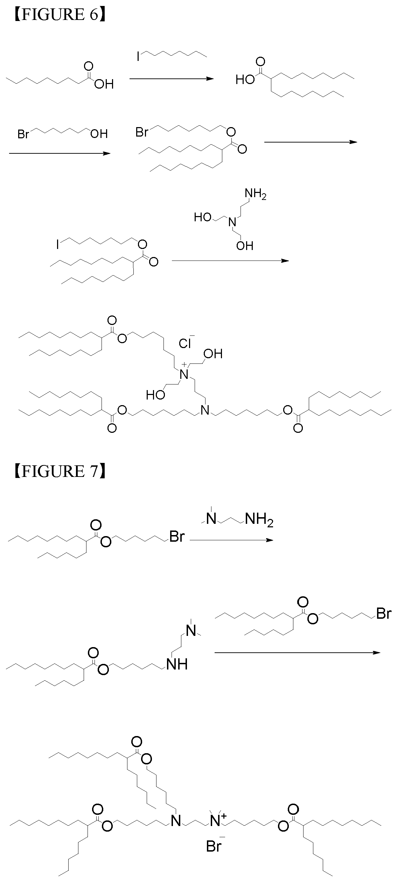

The compound of the following formula G was prepared according to the synthesis scheme shown in Figure 6.

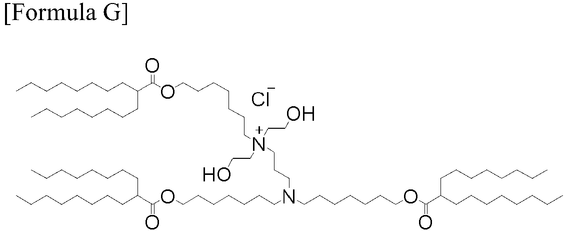

### (1) Synthesis of 2-octyldecanoic acid

In a 1000 mL 3-neck RBF, nonanoic acid (60.00 g, 348.00 mmol, 1.00 eq) and THF (100 mL) were placed under N₂, 0°C conditions. Lithium diisopropylamide (LDA) (2 M, 383.00 mL, 2.20 eq) was added to the mixture under N₂, 0°C conditions and the mixture was stirred at 0°C for 30 minutes. Subsequently, 1-iodooctane (92.00 g, 383.00 mmol, 1.00 eq) was added at room temperature and stirred at 45°C for 16 hours. The reaction mixture was quenched by adding 1 N HCl (1 L), extracted with EtOAc (2 L), dried with Na₂SO₄, filtered, and concentrated to obtain the residue. After purging with N₂ three times, the solution was stirred at 45°C for 16 hours. The residue was purified by silica column chromatography (petroleum ether:EtOAc=100:1→10:1) to obtain 2-octyldecanoic acid (67.00 g, 236.00 mmol, 67.6% yield) in a form of yellow oil.

### (2) Synthesis of 7-bromoheptyl 2-octyldecanoate

In a 250 mL 3-neck RBF, 7-bromoheptan-1-ol (5.14 g, 26.40 mmol, 1.50 eq) dissolved in toluene (50 mL), octyldecanoic acid (5.00 g, 17.60 mmol, 1.00 eq) and H₂SO₄ (259 mg, 2.64 mmol, 0.15 eq) were placed, and the mixture was purged with N₂ three times. The mixture was stirred at 120°C for 16 hours and concentrated under reduced pressure to obtain the residue. The residue was purified by silica column chromatography (petroleum ether:EtOAc=10:1→1:100) to obtain 7-bromoheptyl 2-octyldecanoate (5.50 g, 11.90 mmol, 67.8% yield) in a form of yellow oil.

### (3) Synthesis of 7-iodoheptyl 2-octyldecanoate

In a 250 mL 3-neck RBF, 7-bromoheptyl 2-octyl decanoate (5.50 g, 11.90 mmol, 1.00 eq) was placed, and ACN (55 mL) was added thereto. Then, KI (3.96 g, 23.80 mmol, 2.00 eq) was added, and the mixture was purged with N₂ three times. The mixture was stirred at 90°C for 24 hours, filtered, and concentrated under reduced pressure to obtain 7-iodoheptyl 2-octyl decanoate (5.00 g, 9.83 mmol, 82.5% yield) in a form of yellow oil.

### (4) Synthesis of the compound of formula G

In a 250 mL 3-neck RBF, 7-iodoheptyl 2-octyldecanoate (5.00 g, 9.83 mmol, 4.00 eq) dissolved in ACN (50 mL), 2,2'-((3-aminopropyl)azandyl)bis(ethanol-1-ol) (399.00 mg, 2.46 mmol, 1.00 eq), and K₂CO₃ (42.50 mg, 0.31 mmol, 1.10 eq) were placed, and the mixture was purged with N₂ three times. The solution was stirred at 80°C for 16 hours, filtered, and concentrated under reduced pressure to obtain the residue. The residue was purified by prep-HPLC (column: C1 250×80 mm, 10 µm; mobile phase: [HCl aqueous solution-ACN]; gradient: 60%-90% for 25 minutes) to obtain the compound of formula G (0.25 g, 0.19 mmol, 99.2% yield) in a form of gray oil.

¹H NMR (400 MHz, CHLOROFORM-*d*): *δ* 5.04 - 4.62 (m, 2H), 4.06 (t, *J*=6.8 Hz, 6H), 3.95 - 3.86 (m, 2H), 3.70 - 3.61 (m, 4H), 3.35 - 3.21 (m, 6H), 2.70 (br s, 2H), 2.61 (br s, 4H), 2.31 (tt, *J*=5.4, 8.8 Hz, 3H), 1.85 - 1.69 (m, 10H), 1.66 - 1.53 (m, 12H), 1.46 - 1.36 (m, 24H), 1.26 (s, 65H), 0.93 - 0.84 (m, 18H)

### Example 7

The compound of the following formula H was prepared according to the synthesis scheme shown in Figure 7.

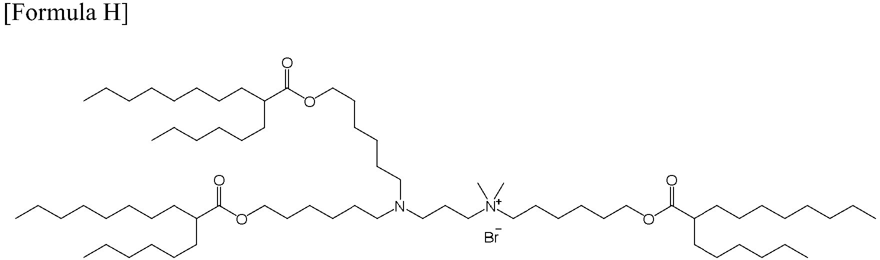

### (1) Synthesis of 6-((3-(dimethylamino)propyl)amino)hexyl 2-hexyldecanoate)

In a 100 mL 3-neck round-bottom flask (RBF), N¹,N¹-dimethylpropane-1,3-diamine (1.0 g, 9.79 mmol, 1.22 mL, 1 eq), 6-bromohexyl 2-hexyl decanoate (4.11 g, 9.79 mmol, 1 eq), and ethanol (20 mL) were placed. N,N-diisopropylethylamine (6.32 g, 48.93 mmol, 8.52 mL, 5.0 eq) was added to this mixture. The mixture was stirred at 90 °C for 3 days. After cooling to room temperature, silica (1 g) was added to the mixture, and the solvent was removed under vacuum. The residue was purified using a silica column with petroleum ether:EtOAc=1:1→2:1 to obtain 6-((3-(dimethylamino)propyl)amino)hexyl 2-hexyl decanoate) (1.08 g, 25.0% yield), in a form of pale yellow oil.

### (2) Synthesis of the compound of formula H

In a 50 mL 3-neck RBF, 6-((3-(dimethylamino)propyl)amino)hexyl 2-hexyl decanoate) (1.08 g, 2.45 mmol, 1 eq), 6-bromohexyl 2-hexyl decanoate (1.03 g, 2.45 mmol, 1 eq) and ethanol (10 mL) were placed. N,N-diisopropylethylamine (633.40 mg, 4.90 mmol, 853.63 µL, 2 eq) was added to the mixture, and the mixture was stirred at 90 °C for 3 days. After cooling to room temperature, silica (1 g) was added to the mixture, and the solvent was removed under vacuum. The residue was purified using a silica column with petroleum ether:EtOAc=3:1→1:1 to obtain the compound of formula H (61.94 mg, 2.3% yield) in a form of pale yellow oil.

¹H NMR (400 MHz, CHLOROFORM-*d*): *δ* 4.07-4.04 (m, 4H), 3.45 (td, 2H), 3.28 (s, 6H), 3.08 (br, 4H), 2.72 (br, H), 2.33-2.27 (m, 3H), 1.84 (br, 6H), 1.68-1.45 (m, 20H), 1.45-1.42 (m, 18H), 1.32-1.25 (m, 62H), 0.89 (t, 18H)

### [Preparation Examples of composition for drug delivery]

### 1. Raw material preparation

According to the following table, the materials required for preparing the formulation were dissolved in each dilution solvent and prepared at the required concentration. When dissolving, the materials were kept at room temperature and then the solvent was added to dissolve them.

| | **Materials** | **Concentration for use** |
|---|---|---|
| 1 | mRNA | 1 mg/mL |
| 2 | Each compound of formulas A to H | 10 - 20 mg/mL |
| 3 | DMG-PEG (1,2-dimyristoyl-rac-glycero-3-methoxypolyethylene glycol-2000) | 5 - 10 mg/mL |
| 4 | Dioleoyl-phosphatidyl-ethanolamine (DOPE) | 10 - 20 mg/mL |
| 5 | Cholesterol | 10 - 20 mg/mL |

### 2. Raw material mixing

The required amounts of the raw materials were taken and mixed in order to meet the N/P ratio (amine group of lipid/phosphate group of mRNA) of 6 and each compound of formulas A to H:DOPE:Cholesterol:DMG-PEG=50:10:38.5:1.5. Ethanol was added to the ethanol layer so that the molecular total of all raw materials was within 12.5 mM, and the aqueous phase and ethanol phase were mixed maintaining a volume ratio of 3:1. After the mixing, to reduce the total ethanol content, buffer exchange was performed as follows: The mixture solution was concentrated by centrifugation at 4,000 rpm using an Amicon-Ultra tube filter (Merk Millipore, UFC505096 or UFC805024, pore size: 50K, volume: 0.5 mL or 4 mL or 15 mL), then diluted with PBS and centrifuged to concentrate. This process was repeated to exchange the buffer.

The more concrete procedure is as follows:
1) Two autoclaved tubes were prepared (tubes (A) and (B)).
2) In tube (A), each compound of formulas A to H, DOPE, cholesterol and DMG-PEG in molar quantities calculated according to the experimental conditions were sequentially added and mixed by vortexing.
3) In the ethanol phase, when necessary, ethanol was added so that the molecular total of all raw materials was within 12.5 mM.
4) In tube (B), mRNA and 20 mM sodium acetate buffer (pH 4.6) were mixed. At that time, the ratio was calculated and added so that the aqueous phase was total three times the amount of the ethanol phase.
5) Mixing of Tube (A) and Tube (B) was performed using a Microfluidics (Ignite, Precision Nanosystem) device. Microfluidics operating conditions were FRR (Flow Rate Ratio) of C:R=3:1 and TRR (Total Flow Rate) of 12 mL/min.
6) The resulting mixture from step 5) was centrifuged at 4,000 rpm using an Amicon-Ultra tube filter (50K) for concentration, and the process of dilution with PBS and concentration by centrifugation was repeated to remove excess ethanol, and then concentrated to a final x mg/ml (theoretical concentration).
7) Once the formulation was concentrated to the desired concentration, it was sterilized using a 0.22 µm pore size filter.

### 3. Evaluation of the properties of the formulation

1) For the prepared formulation, particle characteristics (i.e., Zeta-average particle size (Z-average), polydispersity index (PDI), and zeta-potential) were confirmed using a particle size analyzer (Dynamic Light Scattering, DLS), and the results are shown in Table 1 below.
2) For the prepared formulation, mRNA encapsulation efficiency was confirmed through Ribo-green assay, and the results are shown in Table 1 below.

**[Table 1]**

| **Lipid compound** | **Z-average (nm)** | **PDI (PI)** | **Zeta-potential (mV)** | **Encapsulation efficiency (%)** |
|---|---|---|---|---|
| Formula A | 155.4 ± 0.32 | 0.08 ± 0.03 | -1.85 ± 0.61 | 97.11 |
| Formula B | 150.7 ± 0.21 | 0.28 ± 0.05 | -3.0 ± 0.51 | 97.8 |
| Formula C | 159.3 ± 0.32 | 0.20 ± 0.04 | -6.6 ± 0.56 | 97.6 |
| Formula D | 152.1 ± 0.12 | 0.12 ± 0.01 | -1.8 ± 0.66 | 99.2 |
| Formula E | 153.2 ± 0.22 | 0.13 ± 0.02 | 1.6 ± 0.33 | 98.8 |
| Formula F | 167.2 ± 0.36 | 0.07 ± 0.05 | -1.0 ± 0.22 | 99.1 |
| Formula G | 143.8 ± 0.19 | 0.16 ± 0.03 | -1.2 ± 0.36 | 97.4 |
| Formula H | 155.4 ± 0.32 | 0.08 ± 0.03 | -1.85 ± 0.61 | 97.11 |

## Claims

1. A cationic lipid having a structure represented by the following formula 1: 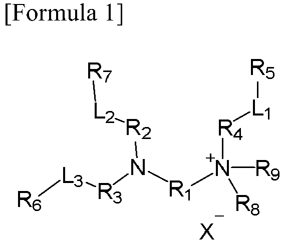 wherein, in the above formula 1,
R₁ is a substituted or unsubstituted alkylene group,
each of R₂, R₃ and R₄ is independently a substituted or unsubstituted alkylene group,
each of R₅, R₆ and R₇ is independently a substituted or unsubstituted, saturated or unsaturated monovalent hydrocarbon group,
each of R₈ and R₉ is independently a substituted or unsubstituted alkyl group or carbocyclic group, or is independently -R₁₀-(L₄)ₙ-R₁₁,
each R₁₀ is independently a substituted or unsubstituted alkylene group,
each R₁₁ is independently a substituted or unsubstituted, saturated or unsaturated monovalent hydrocarbon group,
each of L₁, L₂, L₃ and L₄ is independently selected from the group consisting of -C(O)O-, -OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, alkenylene, arylene, and heteroarylene, wherein L' is a direct bond, alkylene or alkenylene, and each R' is independently selected from the group consisting of hydrogen atom, alkyl and alkenyl,
n is 0 or 1, and
X⁻ is a pharmaceutically acceptable monovalent anion.

2. The cationic lipid according to claim 1, wherein
R₁ is a substituted or unsubstituted C₁₋₆ alkylene group,
each of R₂, R₃ and R₄ is independently a substituted or unsubstituted C₃₋₁₂ alkylene group,
each of R₅, R₆ and R₇ is independently a substituted or unsubstituted, saturated or unsaturated monovalent C₃₋₂₀ hydrocarbon group,
each of R₈ and R₉ is independently a substituted or unsubstituted C₁₋₆ alkyl group or C₃₋₆ carbocyclic group, or is independently -R₁₀-(L₄)ₙ-R₁₁,
each R₁₀ is independently a substituted or unsubstituted C₃₋₁₂ alkylene group,
each R₁₁ is independently a substituted or unsubstituted, saturated or unsaturated monovalent C₃₋₂₀ hydrocarbon group,
each of L₁, L₂, L₃ and L₄ is independently selected from the group consisting of -C(O)O-, -OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, C₂₋₆ alkenylene, C₆₋₂₀ arylene, and C₃₋₂₀ heteroarylene, wherein L' is a direct bond, C₁₋₁₃ alkylene or C₂₋₁₃ alkenylene, and each R' is independently selected from the group consisting of hydrogen atom, C₁₋₁₈ alkyl and C₂₋₁₈ alkenyl,
n is 0 or 1, and
X⁻ is a pharmaceutically acceptable monovalent anion of inorganic acid or organic acid.

3. The cationic lipid according to claim 1, wherein
R₁ is a substituted or unsubstituted C₃₋₄ alkylene group,
each of R₂, R₃ and R₄ is independently a substituted or unsubstituted C₆₋₈ alkylene group,
each of R₅, R₆ and R₇ is independently a substituted or unsubstituted, saturated or unsaturated monovalent C₅₋₁₅ hydrocarbon group,
each of R₈ and R₉ is independently a substituted or unsubstituted C₁₋₂ alkyl group, or is independently -R₁₀-(L₄)ₙ-R₁₁,
each R₁₀ is independently a substituted or unsubstituted C₆₋₈ alkylene group,
each R₁₁ is independently a substituted or unsubstituted, saturated or unsaturated monovalent C₅₋₁₅ hydrocarbon group,
each of L₁, L₂, L₃ and L₄ is independently selected from the group consisting of -C(O)O-, -OC(O)-, -C(O)N(R')-, -N(R')C(O)-, -P(O)(OR')O-, -S-S-, and C₂₋₅ alkenylene, wherein each R' is independently selected from the group consisting of hydrogen atom and C₁₋₆ alkyl,
n is 0 or 1, and
X⁻ is F⁻, Cl⁻, Br⁻, I⁻, nitrate anion, benzoate anion, methanesulfonate anion, acetate anion (CH₃COO⁻), or trihaloacetate anion (CF₃COO⁻).

4. The cationic lipid according to claim 1, wherein the cationic lipid is one having a structure selected from the following formulas A to Q:
| Formula | Structure |
|---|---|
| A | 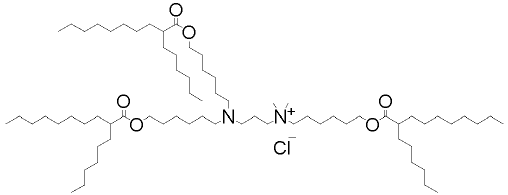 |
| B | 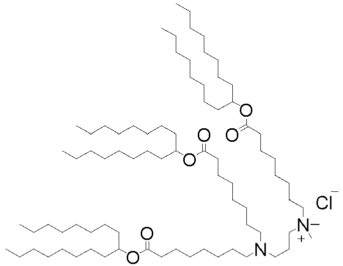 |
| C | 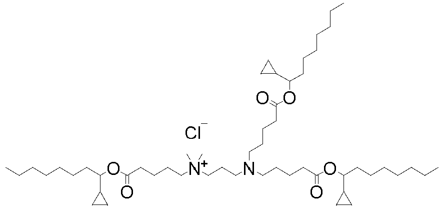 |
| D | 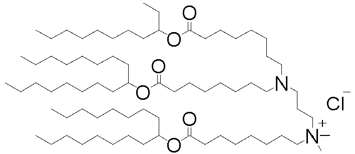 |
| E | 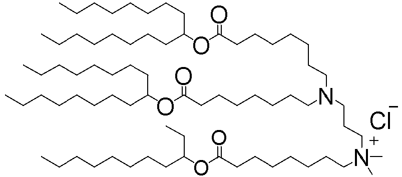 |
| F | 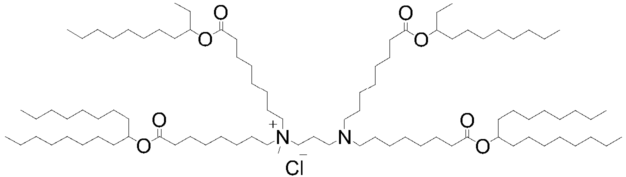 |
| G | 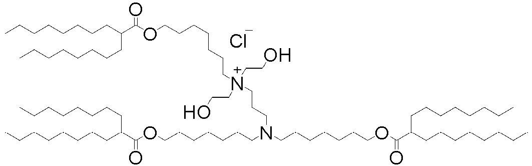 |
| H | 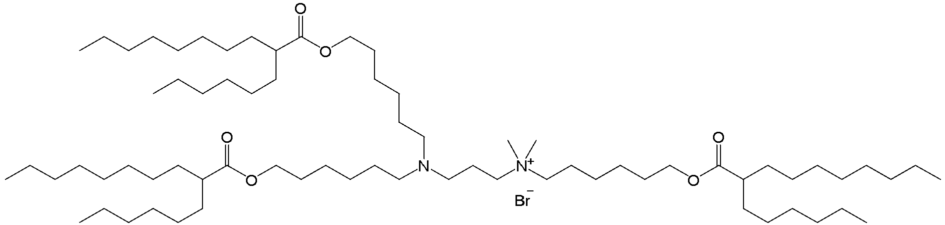 |
| I | 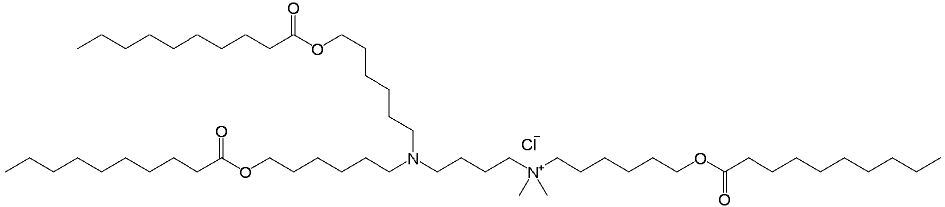 |
| J | 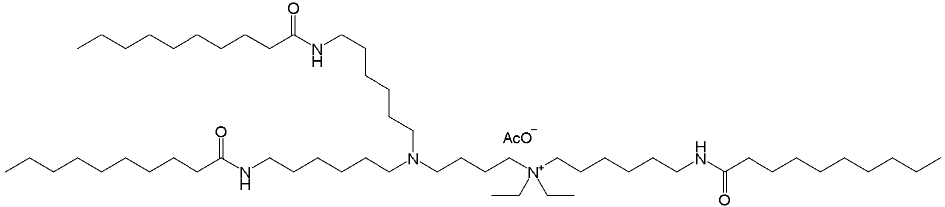 |
| K | 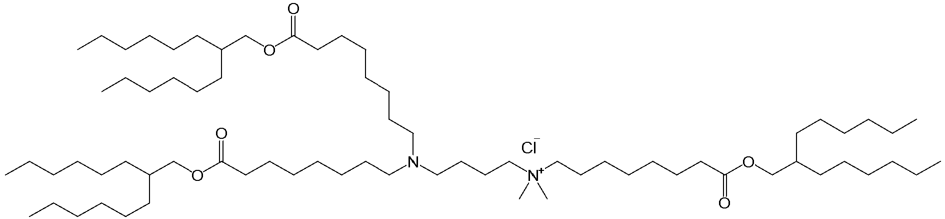 |
| L | 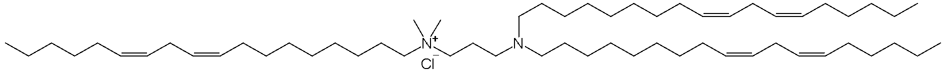 |
| M | 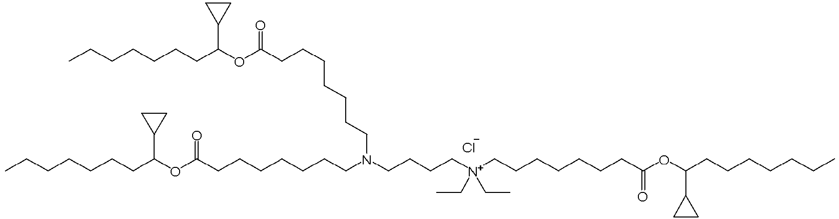 |
| N | 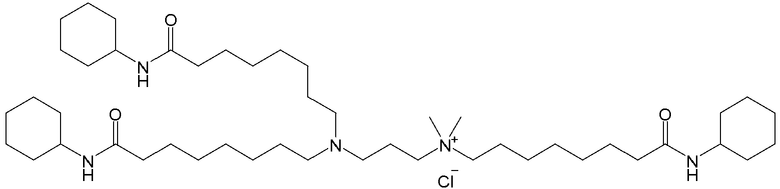 |
| O | 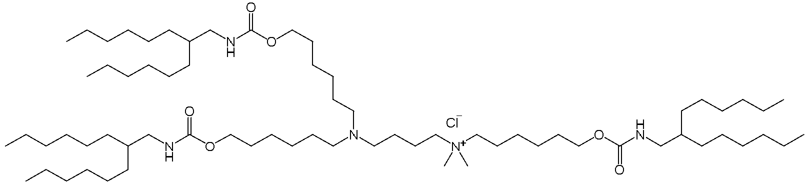 |
| P | 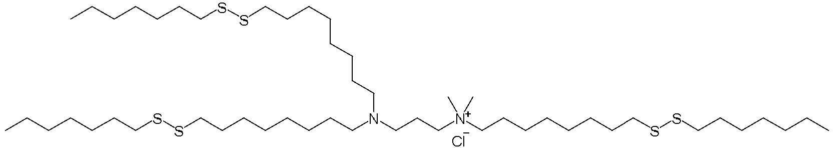 |
| Q | 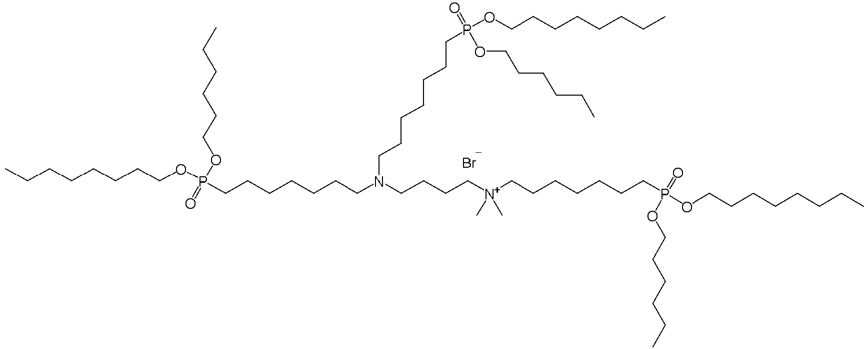 |

5. A method for preparing a cationic lipid having a structure represented by formula 1-1, comprising a step of reacting a compound of formula (a) with a compound of formula (b):
[Formula (a)] R₆-L₃-R₃-X
[Formula (b)] H₂N-R₁-N(-R₈)(-R₉) 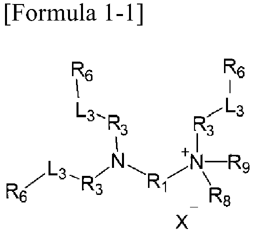
wherein,
R₁ is a substituted or unsubstituted alkylene group,
each R₃ is independently a substituted or unsubstituted alkylene group,
each R₆ is independently a substituted or unsubstituted, saturated or unsaturated monovalent hydrocarbon group,
each of R₈ and R₉ is independently a substituted or unsubstituted alkyl group or carbocyclic group,
each L₃ is independently selected from the group consisting of -C(O)O-, -OC(O)-, - OC(O)-L'-C(O)O-, -C(O)N(R')-, -N(R')C(O)-, -C(O)-, -C(S)-, -C(S)S-, -SC(S)-, -CH(OH)-, - P(O)(OR')O-, -S(O)₂-, -S-S-, alkenylene, arylene, and heteroarylene, wherein L' is a direct bond, alkylene or alkenylene, and each R' is independently selected from the group consisting of hydrogen atom, alkyl and alkenyl, and
X⁻ is a pharmaceutically acceptable monovalent anion.

6. A method for preparing a cationic lipid having a structure represented by formula 1-2 or formula 1-3, comprising steps of: (1) reacting a compound of formula (a) with a compound of formula (b) to obtain a compound of formula (c); and (2) reacting a compound of formula (c) with a compound of formula (d):
[Formula (a)] R₆-L₃-R₃-X
[Formula (b)] H₂N-R₁-N(-R₈)(-R₉)
[Formula (c)] (R₆-L₃-R₃-)NH-R₁-N⁺(-R₈)(-R₉)(-R₃-L₃-R₆)
[Formula (d)] R₇-L₂-R₂-X 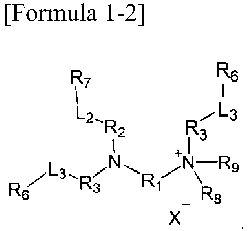 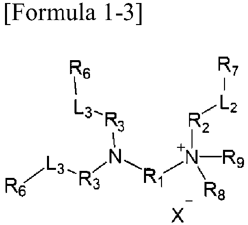
wherein,
R₁ is a substituted or unsubstituted alkylene group,
each of R₂ and R₃ is independently a substituted or unsubstituted alkylene group,
each of R₆ and R₇ is independently a substituted or unsubstituted, saturated or unsaturated monovalent hydrocarbon group,
each of R₈ and R₉ is independently a substituted or unsubstituted alkyl group or carbocyclic group,
each of L₂ and L₃ is independently selected from the group consisting of -C(O)O-, - OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, alkenylene, arylene, and heteroarylene, wherein L' is a direct bond, alkylene or alkenylene, and each R' is independently selected from the group consisting of hydrogen atom, alkyl and alkenyl, and
X⁻ is a pharmaceutically acceptable monovalent anion.

7. A method for preparing a cationic lipid having a structure represented by formula 1-4, comprising steps of: (1) reacting a compound of formula (a) with a compound of formula (b') to obtain a compound of formula (c'); (2) reacting a compound of formula (c') with a compound of formula (d) to obtain a compound of formula (e); and (3) reacting a compound of formula (e) with a compound of formula (f):
[Formula (a)] R₆-L₃-R₃-X
[Formula (b')] H₂N-R₁-NH₂
[Formula (c')] (R₆-L₃-R₃-)NH-R₁-NH(-R₃-L₃-R₆)
[Formula (d)] R₇-L₂-R₂-X
[Formula (e)] (R₆-L₃-R₃-)(R₇-L₂-R₂-)N-R₁-N(-R₂-L₂-R₇)(-R₃-L₃-R₆)
[Formula (f)] R₈-X 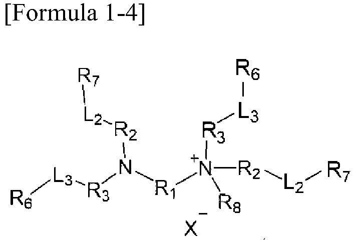
wherein,
R₁ is a substituted or unsubstituted alkylene group,
each of R₂ and R₃ is independently a substituted or unsubstituted alkylene group,
each of R₆ and R₇ is independently a substituted or unsubstituted, saturated or unsaturated monovalent hydrocarbon group,
each R₈ is independently a substituted or unsubstituted alkyl group or carbocyclic group,
each of L₂ and L₃ is independently selected from the group consisting of -C(O)O-, - OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, alkenylene, arylene, and heteroarylene, wherein L' is a direct bond, alkylene or alkenylene, and each R' is independently selected from the group consisting of hydrogen atom, alkyl and alkenyl, and
X⁻ is a pharmaceutically acceptable monovalent anion.

8. A method for preparing a cationic lipid having a structure represented by formula 1-5, comprising steps of: (1) reacting a compound of formula (a) with a compound of formula (b) to obtain a compound of formula (c"); and (2) reacting a compound of formula (c") with a compound of formula (d):
[Formula (a)] R₆-L₃-R₃-X
[Formula (b)] H₂N-R₁-N(-R₈)(-R₉)
[Formula (c")] (R₆-L₃-R₃-)NH-R₁-N(-R₈)(-R₉)
[Formula (d)] R₇-L₂-R₂-X 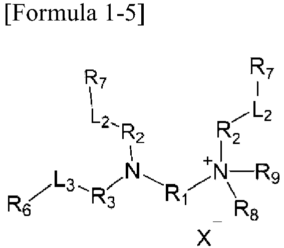
wherein,
R₁ is a substituted or unsubstituted alkylene group,
each of R₂ and R₃ is independently a substituted or unsubstituted alkylene group,
each of R₆ and R₇ is independently a substituted or unsubstituted, saturated or unsaturated monovalent hydrocarbon group,
each of R₈ and R₉ is independently a substituted or unsubstituted alkyl group or carbocyclic group,
each of L₂ and L₃ is independently selected from the group consisting of -C(O)O-, - OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, alkenylene, arylene, and heteroarylene, wherein L' is a direct bond, alkylene or alkenylene, and each R' is independently selected from the group consisting of hydrogen atom, alkyl and alkenyl, and
X⁻ is a pharmaceutically acceptable monovalent anion.

9. A composition for drug delivery comprising the cationic lipid of any one of claims 1 to 4.
